(19) Europäisches Patentamt
European Patent Office
Office européen des brevets

(11) EP 3 010 508 B1

(12) **EUROPEAN PATENT SPECIFICATION**

(45) Date of publication and mention of the grant of the patent:
**24.07.2019 Bulletin 2019/30**

(21) Application number: **14731223.5**

(22) Date of filing: **16.06.2014**

(51) Int Cl.:
***A61K 31/555*** (2006.01) ***A61K 31/40*** (2006.01)
***A61K 31/7068*** (2006.01) ***A61P 35/00*** (2006.01)

(86) International application number:
**PCT/EP2014/062496**

(87) International publication number:
**WO 2014/202497 (24.12.2014 Gazette 2014/52)**

(54) **COMBINATION OF RO5503781, CAPECITABINE AND OXALIPLATIN FOR CANCER THERAPY**

KOMBINATION AUS RO5503781, CAPECITABIN UND OXALIPLANTIN ZUR KREBSTHERAPIE

COMBINAISON DE RO5503781, DE CAPÉCITABINE ET D'OXALIPLATINE POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.06.2013 US 201361836899 P**

(43) Date of publication of application:
**27.04.2016 Bulletin 2016/17**

(73) Proprietor: **F. Hoffmann-La Roche AG**
**4070 Basel (CH)**

(72) Inventors:
- **HIGGINS, Brian**
  **Fresh Meadows, New York 11365 (US)**
- **NICHOLS, Gwen**
  **New York, New York 10128 (US)**
- **PACKMAN, Kathryn E.**
  **Newton, Massachusetts 02461 (US)**

(74) Representative: **Beyermann, Jochen Carl**
**F. Hoffmann-La Roche AG**
**CLP - Patent Department**
**Grenzacherstrasse 124**
**4070 Basel (CH)**

(56) References cited:

- **KRZYSZTOF ZAK ET AL: "Mdm2 and MdmX inhibitors for the treatment of cancer: a patent review (2011 - present)", EXPERT OPINION ON THERAPEUTIC PATENTS, vol. 23, no. 4, 1 April 2013 (2013-04-01), pages 425-448, XP055107012, ISSN: 1354-3776, DOI: 10.1517/13543776.2013.765405**
- **KAREEM KHOURY ET AL: "The p53-MDM2/MDMX axis - A chemotype perspective", MEDCHEMCOMM, vol. 2, no. 4, 1 January 2011 (2011-01-01) , page 246, XP055136675, ISSN: 2040-2503, DOI: 10.1039/c0md00248h**
- **HOFFMAN-LA ROCHE: "A First-In-Human Study of RO5503781 in Patients With Advanced Malignancies, Except Leukemia", INTERNET CITATION, 27 October 2011 (2011-10-27), pages 1-2, XP002696086, Retrieved from the Internet: URL:http://www.clinicaltrials.gov/ct2/show /NCT01462175?term=RO5503781&rank=2 [retrieved on 2013-04-24]**
- **QINGJIE DING ET AL: "Discovery of RG7388, a Potent and Selective p53-MDM2 Inhibitor in Clinical Development", JOURNAL OF MEDICINAL CHEMISTRY, vol. 56, no. 14, 25 July 2013 (2013-07-25) , pages 5979-5983, XP055109745, ISSN: 0022-2623, DOI: 10.1021/jm400487c**

EP 3 010 508 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- Asfar S. Azmi ET AL: "Network Perspectives on HDM2 Inhibitor Chemotherapy Combinations", Current Pharmaceutical Design, 1 February 2011 (2011-02-01), pages 640-652, XP055137179, Netherlands Retrieved from the Internet: URL:http://www.eurekaselect.com/openurl/content.php?genre=article&issn=13816128&volume=17&issue=6&spage=640
- J. CASSIDY: "XELOX (Capecitabine Plus Oxaliplatin): Active First-Line Therapy for Patients With Metastatic Colorectal Cancer", JOURNAL OF CLINICAL ONCOLOGY, vol. 22, no. 11, 13 April 2004 (2004-04-13), pages 2084-2091, XP055136569, ISSN: 0732-183X, DOI: 10.1200/JCO.2004.11.069
- YU HONG LI ET AL: "Phase II study of capecitabine plus oxaliplatin (XELOX) as first-line treatment and followed by maintenance of capecitabine in patients with metastatic colorectal cancer", JOURNAL OF CANCER RESEARCH AND CLINICAL ONCOLOGY, SPRINGER, BERLIN, DE, vol. 136, no. 4, 24 September 2009 (2009-09-24), pages 503-510, XP019803226, ISSN: 1432-1335

## Description

### FIELD OF THE INVENTION

The present invention relates to a method of cancer therapy by administering a pharmaceutical composition containing (i) a compound of formula

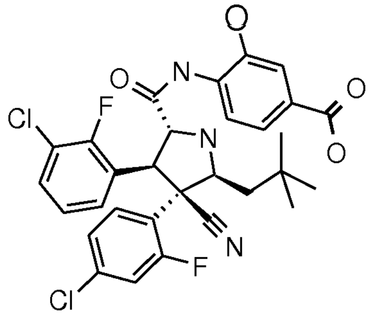

(A)

4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) which is an antagonist of the p53/MDM2 interaction , (ii) a pharmaceutical composition containing capecitabine which is a fluoropyrimidine carbamate with antineoplastic activity and (iii) a pharmaceutical composition containing oxaliplatin which is a platinum based antineoplastic agent. Capecitabine is an orally administered systemic prodrug of 5'-doexy-5-fluorouridine (5'-DFUR) which is converted to 5-fluorouracil intracellularly, an antineoplastic agent. The invention is also directed to the pharmaceutical product comprising components, as indicated in item (i), (ii) and (iii) above, for use in the combined, sequential or simultaneous, treatment of cancer.

Capecitabine is marketed in the United States under the brand name Xeloda®. The chemical name for capecitabine is 5'-deoxy-5-fluoro-N-[(pentyloxy)-carbonyl]-cytidine and has the following structural formula:

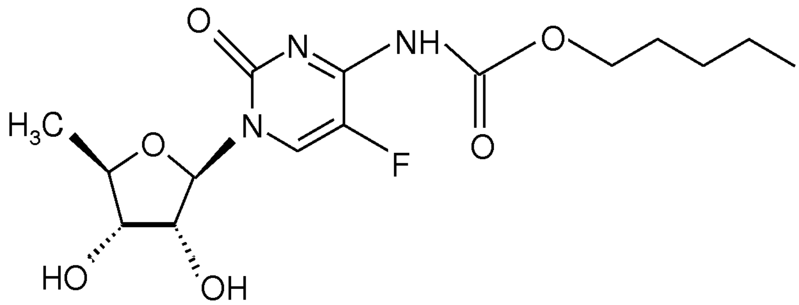

Capecitabine is covered in US patents, including US Pat. No. 4,966,891 and 5,472,949. Improved methods for the manufacture of capecitabine are also taught by US Pat. No. 5,453,497 and 5,476,932, and application USSN 60/532,266, filed December 22, 2003.

Compound A is disclosed in WO2011/098398 and US Patent No. 8,354,444. Specific pharmaceutical preparations comprising Compound A are also disclosed in International Patent Application No. PCT/EP2014/050974.

Oxaliplatin is a platinum based antineoplastic agent having the structural formula

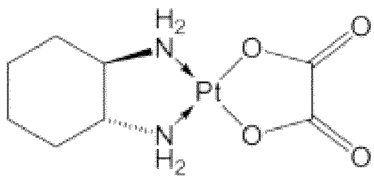

and is sold by Sanofi-Aventis under the trademark Eloxatin™. Dosages, in particular also the maximum tolerated dose (MTD), dosage schedules are publicly available to the person of skill in the art, i.e. a clinical physician.

The invention is also directed to a kit containing both of the above compositions.

## BACKGROUND OF THE INVENTION

p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p161NK4/p19ARF locus, for instance, have been shown to affect MDM2 protein degradation. Inhibition of the p53-MDM2 interaction in tumor cells with activation of the p53-MDM2 pathway should lead to accumulation of p53, cell cycle arrest and/or apoptosis. The feasibility of p53/MDM2 antagonism as a strategy has been shown by the use of different macromolecular tools for inhibition of MDM2-p53 interaction (e.g. antibodies, antisense oligonucleotides, peptides).

Azmi et al. (current pharmaceutical design, 2011, 17,640-652) discloses that HDM2 inhibitors can act synergistically with platinum drugs, displaying an enhanced antitumor activity.

## DETAILED DESCRIPTION OF THE INVENTION

In one embodiment, the present invention relates to a pharmaceutical product comprising a) as a first component a pharmaceutical composition comprising as an active ingredient a therapeutically effective amount of 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) or a pharmaceutically acceptable salt, ester or prodrug of said compound; b) a second component comprising a pharmaceutical composition comprising a therapeutically effective amount of capecitabine; and c) a third component comprising a pharmaceutical composition comprising a therapeutically effective amount of oxaliplatin for the combined, sequential or simultaneous, treatment of cancer, in particular solid tumors such as colon, colorectal, breast and lung cancer.

Within this embodiment, the pharmaceutical compositions according to b) and c) are preferably the medicaments marketed as Xeloda™ and Eloxatin™, respectively.

In another embodiment, this combination of chemotherapeutic compounds is particularly useful in the treatment of colon cancer.

In another embodiment, the present invention relates to a method of treating a patient suffering with cancer comprising administering to the patient, either concomitantly or sequentially, a first component consisting of a pharmaceutical composition containing as an active ingredient a therapeutically effective amount of a compound of 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) or a pharmaceutically acceptable salt or ester of said compound, a second component consisting of a pharmaceutical composition containing a therapeutically effective amount of capecitabine and a third component consisting of a pharmaceutical composition containing oxaliplatin.

It was unexpectedly found that administration of the three components in accordance with the present invention results in improved antineoplastic effects that are significantly superior to the results obtained with each compound alone. Namely, administration of the three components in accordance with the present invention resulted in an improved therapeutic index (that is, superior efficacy) in comparison to either component alone without a significant increase in toxicity. Alternatively the invention permits reduction of the amount of at least one component (in comparison the amount typically given in monotherapy) while retaining a desirable therapeutic index. In preferred embodiments, the amount of the 3 components (in comparison the amount typically given in monotherapy) is reduced affording reduced toxicity while still retaining a desirable therapeutic index.

In another embodiment, the present invention provides a pharmaceutical product, or relates to a method of treating a patient suffering with cancer, characterized by administering Compound A in an amount of from about 800 to 3200 mg/day or 400 to about 3200 mg/day, or from about 400 to about 1600 mg/day, or from about 1000 to about 2500 mg/day, or from about 1250 to about 1800 mg/day, for an administration period of up to about 7 days, preferably once per week or up to about 5 days, more preferably once per week, on days 1-3, or on days 1-5, of a 28 day treatment cycle, followed by a rest period of from about 21 to about 23 days, preferably up to about 23 days together with, in combination with Compound A, capecitabine in an amount of about 800-1500 $mg/m^2$ twice daily over a period of 14 days and oxaliplatin in an amount of about 75- 130 $mg/m^2$ once every three weeks .

The course of a preferred cycle is about 28 days, though cycles anywhere between about 14 and about 28 days are contemplated. This treatment cycle is repeated for as long as the tumor remains under control and the regimen is clinically tolerated.

Dosages of Compound A can be applied either as a body surface area ("BSA") adapted dose ($mg/m^2/day$) or following flat dosing (mg/day). Compound A may be administered as a single dose daily or divided into multiple daily doses.

A patient's body measurement in square meters ("$m^2$") typically ranges from about 1.4 $m^2$ to about 2.2 $m^2$, Thus, the total amount of Compound A to be delivered in a treatment cycle (mg) using a BSA adapted dose would be calculated as follows:

$$[\text{Dose intensity}(mg/m^2/week)] \times [BSA(m^2)] \times [\text{number of weeks in treatment cycle}].$$

For capecitabine in combination with Compound A the preferred dose is 800-1500 mg/$m^2$ twice daily over a period of 14 days.

In an embodiment, Compound A is administered daily for about 5 days, on days 1-5 of a weekly treatment cycle, followed by a rest period of 23 days ("5+/23-"). Compound A is administered daily, either once or twice (bid) daily, preferably once daily. The compound is administered to the patient in an oral unit dosage form, most preferably in tablet form.

Preferably, the 5 day per week treatment schedule is repeated every twenty-eight days, or as soon as permitted by recovery from toxicity, for so long as the tumor is under control or regressing and the patient tolerates the regimen. Preferably, these treatment cycles are repeated for a total of up to about 12 cycles.

In an embodiment, Compound A is administered daily in an amount from about 400 to about 3000 mg/day for up to about 3 days on days 1-5 of a weekly 28 day cycle.

In an embodiment, Compound A is administered daily in an amount from about 400 to about 1500 mg/day for up to about 5 days on days 1-5 of a weekly 28 day cycle.

In an embodiment, Compound A is administered daily in an amount from about 800 to about 3000 mg/day for up to about 5 days on days 1-5 of a weekly 28 day cycle.

In another embodiment, Compound A is administered daily in an amount from about 800 to about 3200 mg/day weekly on days 1, 7, 15 of a weekly 28 day cycle.

In another embodiment, Compound A is administered daily in an amount from about 1250 to about 1800 mg/day weekly on days 1, 7, 15 of a weekly 28 day cycle.

In an embodiment, Compound A is administered daily in an amount from about 400 to about 1600 mg/day for up to about 7 days on days 1-7 of a weekly 28 day cycle.

In another embodiment, the present invention provides a pharmaceutical product comprising Compound A, capecitabine and oxaliplatin in the dosages and dosage schedules according to treatment groups 9 and 10 of Example 1 as disclosed herein.

In another embodiment, the present invention provides a) Compound A or a pharmaceutically acceptable salt, ester or prodrug thereof; b) capecitabine; and c) oxaliplatin for the preparation of a medicament for the combined, sequential or simultaneous, treatment of cancer, in particular solid tumors such as colon, colorectal, breast and lung cancer. Within this embodiment, the pharmaceutical compositions according to b) and c) are preferably the medicaments marketed as Xeloda™ and Eloxatin™, respectively.

Abbreviations used herein are as follows:

| | |
|---|---|
| x | times |
| po | orally |
| bid | twice daily |
| wk | week |
| qd | once daily |
| qdx5 | once daily for five days |
| qweekly or 1x/wk | once a week |
| BWL | body weight loss |
| SD | standard deviation |

The LoVo cell line was selected for implantation in mice as it is has activation of the p53-MDM2 pathway but lacks MDM2 amplification or overexpression, which therefore is felt to be more reflective of clinical reality of the colorectal cancer patient population of interest.

## EXAMPLES

Example 1

Compound A and the capecitabine/oxaliplatin formulations were as follows. If not explicitly indicated otherwise, the amounts provided below are concentrations [mg/ml]. Compound A is used at concentrations of 10 mg/ml and 12.5

mg/ml. Capecitabine is used at concentrations of 12.5 mg/ml, 25mg/ml and 50mg/ml. Oxaliplatin is dosed at 5mg/kg. The vehicle solutions for compound A and capecitabine are as follows:

Vehicle solution for Compound A

Klucel LF: 20.0 mg/mL
Tween 80: 1.0 mg/mL
Methylparaben: 0.9 mg/mL
Propylparaben: 0.1 mg/mL
Water for Injection: Qs to 1.0ml

Vehicle solution for oral suspension of capecitabine

Klucel LF: 20.0 mg/mL
Polysorbate 80: 1.0 mg/mL
Methylparaben: 0.9 mg/mL
Propylparaben: 0.1 mg/mL
Purified Water: Qs to 1.0 ml

Compound A is provided as powder for constitution prior to dosing. The powder can be kept at room temperature. The vehicle solution is prepared immediately prior to constitution or, if prepared earlier, kept at 2-8 °C.

Constitution instructions:

1. Take out the vehicle from the storage refrigerator.
2. Take one vial of the Compound A Powder for Constitution. Add the amount of vehicle indicated on the label. For easier constitution, it is recommended to add a small portion to wet the powder first and then add the remaining amount of vehicle. Mix the suspension using a magnetic bar at low speed for 30 minutes or vortex it till the powder is fully wet and suspended before dosing. If needed, use a spatula to help wet the powder periodically during the course of mixing.
3. Continue mixing while withdraw for dosing.

The capecitabine suspension should be stored at 2-8 °C after preparation. While preparing this suspension good mixing (stir for at least 30 min) is required. Stirring should be continued while dosing the suspension. Dosing of oxaliplatin can be effected using commercially available drug substance, or according to instruction well known to the person of skill in the art.

The following treatment groups were used in the study

1. Vehicle Control qd x 5 po + qd x 14 po
2. Compound A 80 mg/kg qd x 5 po
3. Compound A 100 mg/kg qweekly x 3 po
4. Capecitabine 200 mg/kg qd x 14 po
5. Oxaliplatin 5mg/kg qweekly ip × 2
6. Capecitabine 200 mg/kg qd× 14po+ Oxaliplatin 5mg/kg qweekly ip × 2
7. Compound A 80 mg/kg qd x 5 + Capecitabine 200 mg/kg qd x 14
8. Compound A 100 mg/kg qweekly × 3 + Capecitabine 200 mg/kg qd x 14
9. Compound A 80 mg/kg qd × 5 + Capecitabine 200 mg/kg qd x 14 + Oxaliplatin 5mg/kg qweekly ip × 2
10. Compound A 100 mg/kg qweekly x 3 + Capecitabine 200 mg/kg qd x 14 + Oxaliplatin 5mg/kg qweekly ip × 2

**Table 1: Treatment Results**

| Group | Schedule | Mean Tumor Volume | %T/C (EOS) | %Inhibition (EOS) | %Increased Life Span |
|---|---|---|---|---|---|
| Vehicle | qdx5 | 129.71 | ------- | --------- | -------- |
| | qdx14 | | ------- | --------- | -------- |
| | qweekly×2ip | | ------- | --------- | -------- |

(continued)

| | | | | | |
|---|---|---|---|---|---|
| Cpd. A | | | | | |
| 80 mg/kg | qdx5 | 130.73 | 38 | 64 | 21 |
| 100mg/kg | qwkly×3 | 130.23 | 40 | 60 | 21 |
| Capecitabine | | | | | |
| 200mg/kg | qdx14 | 129.07 | 25 | 75 | 36 |
| Oxaliplatin | | | | | |
| 5mg/kg | qweekly×2ip | 130.60 | 77 | 23 | 0 |
| Capecitabine + Oxaliplatin | | | | | |
| 200 mg/kg<br>5mg/kg | qd×14 +<br>qweekly×2 | 128.37 | 11 | 89 | 62 |
| Cpd A + Capecitabine | | | | | |
| 80mg/kg +<br>200mg/kg | qdx5<br>qdx14 | 130.12 | 7 | 93 | 41 |
| 100mg/k +<br>200 mg/kg | qwkly×3<br>qd × 14 | 130.37 | 6 | 94 | 71 |
| Cpd A + Capecitabine + Oxaliplatin | | | | | |
| 80mg/kg+<br>200mg/kg+<br>5mg/kg | qd×5<br>qd×14<br>qwkly×2 | 130.59 | -10 | regression | 100 |
| Cpd A + Capecitabine + Oxaliplatin | | | | | |
| 100mg/kg+<br>200mg/kg+<br>5mg/kg | qwkly3<br>qd × 14<br>qwkly | 133.09<br>×2 | -11 | regression | 124 |
| CpdA and Capecitabine dosing is per os. Oxaliplatin is ip. | | | | | |

Discussion of Results

This study was conducted to test the addition of Cpd A to capecitabine plus oxaliplatin ("Xelox") and see how activity compares to the Xelox and Cpd A/capecitabine doublets. The growth inhibition (TGI) for both capecitabine + Cpd A doublets were statistically equivalent to the TGI in the Xelox doublet, however the survival in the Cpd A 100 mg/kg doublet was better than Xelox while that in the 80 mg/kg Cpd A doublet was not. The TGI and survival for both triplets were better than both capecitabine + Cpd A doublets and the Xelox doublet. The TGI and survival for the triplets were statistically equivalent when compared to each other.

These data suggests that MDM2 inhibitor Cpd A can enhance the activity of capecitabine in this preclinical model, particularly using the qweekly Cpd A regimen. In combination with Xelox, either regimen of Cpd A enhances TGI and life span (ILS) in this preclinical model. These data provide preclinical support for various clinical testing scenarios in patients with metastatic p53 wildtype colorectal cancer.

## Claims

1. A pharmaceutical product comprising a) as a first component a pharmaceutical composition comprising as an active ingredient a therapeutically effective amount of 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid (Compound A) or a pharmaceutically acceptable salt, ester of said compound; b) a second component comprising a pharmaceutical composition comprising a therapeutically effective amount of capecitabine; and c) a third component comprising a pharmaceutical composition comprising a therapeutically effective amount of oxaliplatin for use in the combined, sequential or simultaneous, treatment of cancer, in particular solid tumors such as colon, colorectal, breast and lung cancer.

2. The product for use according to claim 1 wherein the capecitabine is dosed at about 800 to about 1500 mg/$m^2$ twice daily over a period of 14 days.

3. The product for use according to claim 2 wherein the oxaliplatin is dosed at about 75- 130 mg/$m^2$ every three weeks.

4. The product for use according to claim 3 wherein the dosage of 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2- carbonyl]-amino }-3-methoxy-benzoic acid in an amount from about 400 mg/day to about 1600 mg/day, daily, for up to about 7 days, followed by a rest period of up to about 21 days, said administration starting on the first day of a 28 day treatment cycle.

5. The product for use according to claim 3 wherein the dosage of 4-{[(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2-carbonyl]-amino}-3-methoxy-benzoic acid is in an amount from about 400 mg/day to about 3200 mg/day, daily, for up to about 5 days, followed by a rest period of up to about 23 days, said administration starting on the first day of a 28 day treatment cycle.

6. The product for use according to claim 3 wherein the dosage of 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2-carbonyl]-amino}-3-methoxy-benzoic acid is in an amount from about 800 mg/day to about 3200 mg/day once per week, followed by a rest period of up to about 21 days, said administration starting on the first day of a 28 day treatment cycle.

7. The product for use according to claim 3 wherein 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino}-3-methoxy-benzoic acid is administered in an amount of from about 800 mg/day to about 1500 mg/day.

8. The product for use according to claim 3 wherein 4-{[(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2-carbonyl]-amino}-3-methoxy-benzoic acid is administered in an amount of from about 1000 mg/day to about 2500 mg/day.

9. The product for use according to claim 3 wherein 4-{ [(2R,3S,4R,5S)-4-(4-Chloro-2-fluoro phenyl)-3-(3-chloro-2-fluoro-phenyl)-4-cyano-5-(2,2-dimethyl-propyl)-pyrrolidine-2- carbonyl]-amino}-3-methoxy-benzoic acid is administered in an amount of from about 1250 mg/day to about 1800 mg/day.

10. The product for use according to claim 3 wherein the treatment cycle is repeated every 28 days for up to about 12 cycles.

11. The product for use according to claim 3 wherein the 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid is administered weekly on days 1, 7, 15 of a weekly 28 day cycle.

12. The product for use according to claim 3 wherein 4-{[(2R,3S,4R,5S)-4-(4-Chloro-2-fluorophenyl)-3-(3-chloro-2-fluoro-phenyl)-4-cyano-5-(2,2-dimethyl-propyl)-pyrrolidine-2-carbonyl]-amino}-3-methoxy-benzoic acid is administered once daily for 5 days weekly every 28 days.

13. A kit comprising: (a) a first component containing one or more oral unit dosage forms of an active ingredient comprising capecitabine, (b) a second component containing one or more oral unit dosage forms of an active ingredient comprising oxaliplatin and (c) a third component comprising 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid.

14. The kit of claim 13, wherein the first component contains a sufficient number of units so that a patient can administer about 800 -1500 mg/$m^2$ of capecitabine twice daily over a period of 14 days and the second component contains a sufficient number of doses so that a patient can administer about 75- 130 mg/m2 every three weeks and the third component contains a sufficient number of doses so that a patient can administer about 400-3000 mg per day of 4-{[(2R,3S,4R,5S)-4-(4-Chloro-2-fluoro-phenyl)-3-(3-chloro-2-fluoro-phenyl)-4-cyano-5-(2,2-dimethyl-propyl)-pyrrolidine-2-carbonyl]-amino}-3-methoxy-benzoic acid for a period of up to 5 days.

**Patentansprüche**

1. Pharmazeutisches Produkt, umfassend a) als erste Komponente eine pharmazeutische Zusammensetzung, umfassend als Wirkstoff eine therapeutisch wirksame Menge an 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure (Verbindung A) oder einem pharmazeutisch unbedenklichen Salz, Ester der Verbindung; b) eine zweite Komponente, umfassend eine pharmazeutische Zusammensetzung, umfassend eine therapeutisch wirksame Menge an Capecitabin; und c) eine dritte Komponente, umfassend eine pharmazeutische Zusammensetzung, umfassend eine therapeutisch wirksame Menge an Oxaliplatin, zur Verwendung bei der kombinierten, sequenziellen oder gleichzeitigen Behandlung von Krebs, insbesondere von festen Tumoren wie Kolon-, Dickdarm-, Brust- und Lungenkrebs.

**2.** Produkt zur Verwendung nach Anspruch 1, wobei das Capecitabin zweimal täglich zu etwa 800 bis etwa 1500 $mg/m^2$ über einen Zeitraum von 14 Tagen dosiert wird.

**3.** Produkt zur Verwendung nach Anspruch 2, wobei das Oxaliplatin alle drei Wochen zu etwa 75-130 $mg/m^2$ dosiert wird.

**4.** Produkt zur Verwendung nach Anspruch 3, wobei die Dosierung der 4-{[(2R,3S,4R,5S)-4-(4-Chlor-2-fluorphenyl)-3-(3-chlor-2-fluor-phenyl)-4-cyano-5-(2,2-dimethyl-propyl)-pyrrolidin-2-carbonyl]-amino}-3-methoxybenzoesäure täglich in einer Menge von etwa 400 mg/Tag bis etwa 1600 mg/Tag über bis zu etwa 7 Tagen, gefolgt von einer Ruheperiode von bis zu etwa 21 Tagen, wobei die Verabreichung am ersten Tag eines 28-tägigen Behandlungszyklus beginnt.

**5.** Produkt zur Verwendung nach Anspruch 3, wobei die Dosierung der 4-{[(2R,3S,4R,5S)-4-(4-Chlor-2-fluor phenyl)-3-(3-chlor-2-fluor-phenyl)-4-cyano-5-(2,2-dimethyl-propyl)-pyrrolidin-2-carbonyl]-amino}-3-methoxybenzoesäure täglich in einer Menge von etwa 400 mg/Tag bis etwa 3200 mg/Tag über bis zu etwa 5 Tagen, gefolgt von einer Ruheperiode von bis zu etwa 23 Tagen erfolgt, wobei die Verabreichung am ersten Tag eines 28-tägigen Behandlungszyklus beginnt.

**6.** Produkt zur Verwendung nach Anspruch 3, wobei die Dosierung der 4-{[(2R,3S,4R,5S)-4-(4-Chlor-2-fluor phenyl)-3-(3-chlor-2-fluor-phenyl)-4-cyano-5-(2,2-dimethyl-propyl)-pyrrolidin-2-carbonyl]-amino}-3-methoxy-benzoesäure einmal pro Woche in einer Menge von etwa 800 mg/Tag bis etwa 3200 mg/Tag, gefolgt von einer Ruheperiode von bis zu etwa 21 Tagen erfolgt, wobei die Verabreichung am ersten Tag eines 28-tägigen Behandlungszyklus beginnt.

**7.** Produkt zur Verwendung nach Anspruch 3, wobei die 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure in einer Menge von etwa 800 mg/Tag bis etwa 1500 mg/Tag verabreicht wird.

**8.** Produkt zur Verwendung nach Anspruch 3, wobei die 4-{[(2R,3S,4R,5S)-4-(4-Chlor-2-fluor phenyl)-3-(3-chlor-2-fluor-phenyl)-4-cyano-5-(2,2-dimethyl-propyl)-pyrrolidin-2-carbonyl]-amino}-3-methoxy-benzoesäure in einer Menge von etwa 1000 mg/Tag bis etwa 2500 mg/Tag verabreicht wird.

**9.** Produkt zur Verwendung nach Anspruch 3, wobei die 4-{[(2R,3S,4R,5S)-4-(4-Chlor-2-fluor phenyl)-3-(3-chlor-2-fluor-phenyl)-4-cyano-5-(2,2-dimethyl-propyl)-pyrrolidin-2-carbonyl]-amino}-3-methoxy-benzoesäure in einer Menge von etwa 1250 mg/Tag bis etwa 1800 mg/Tag verabreicht wird.

**10.** Produkt zur Verwendung nach Anspruch 3, wobei der Behandlungszyklus alle 28 Tage über bis zu etwa 12 Zyklen wiederholt wird.

**11.** Produkt zur Verwendung nach Anspruch 3, wobei die 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure wöchentlich an den Tagen 1, 7, 15 eines wöchentlichen 28-tägigen Zyklus verabreicht wird.

**12.** Produkt zur Verwendung nach Anspruch 3, wobei die 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure alle 28 Tage einmal täglich über 5 Tage wöchentlich verabreicht wird.

**13.** Kit umfassend: (a) eine erste Komponente, die eine oder mehrere orale Einheitsdosierungsformen eines Wirkstoffs, umfassend Capecitabin, enthält, (b) eine zweite Komponente, die eine oder mehrere orale Einheitsdosierungsformen eines Wirkstoffs, umfassend Oxaliplatin, enthält, und (c) eine dritte Komponente, umfassend 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure.

**14.** Kit nach Anspruch 13, wobei die erste Komponente eine ausreichende Anzahl Einheiten enthält, um einem Patienten

etwa 800-1500 mg/$m^2$ Capecitabin zweimal täglich über einen Zeitraum von 14 Tagen verabreichen zu können, und die zweite Komponente eine ausreichende Anzahl Dosen enthält, um einem Patienten etwa 75-130 mg/$m^2$ alle drei Wochen verabreichen zu können, und die dritte Komponente eine ausreichende Anzahl Dosen enthält, um einem Patienten etwa 400-3000 mg pro Tag der 4-{[(2R,3S,4R,5S)-4-(4-Chlor-2-fluor-phenyl)-3-(3-chlor-2-fluor-phenyl)-4-cyano-5-(2,2-dimethyl-propyl)-pyrrolidin-2-carbonyl]-amino}-3-methoxy-benzoesäure über einen Zeitraum von bis zu 5 Tagen verabreichen zu können.

## Revendications

1. Produit pharmaceutique comprenant a) une composition pharmaceutique en tant que premier constituant comprenant, comme principe actif, une quantité thérapeutiquement efficace d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque (Composé A) ou d'un sel pharmaceutiquement acceptable, d'un ester dudit composé ; b) un deuxième constituant comprenant une composition pharmaceutique comprenant une quantité thérapeutiquement efficace de capécitabine ; et c) un troisième constituant comprenant une composition pharmaceutique comprenant une quantité thérapeutiquement efficace d'oxaliplatine pour une utilisation dans le traitement combiné, séquentiel ou simultané du cancer, en particulier des tumeurs solides telles que les cancers du côlon, colorectal, du sein et du poumon.

2. Produit pour une utilisation selon la revendication 1, dans lequel la capécitabine est dosée à environ 800 à environ 1 500 mg/$m^2$ deux fois par jour sur une période de 14 jours.

3. Produit pour une utilisation selon la revendication 2, dans lequel l'oxaliplatine est dosé à environ 75 à 130 mg/$m^2$ toutes les trois semaines.

4. Produit pour une utilisation selon la revendication 3, dans lequel la dose d'acide 4-{[(2R,3S,4R,5S)-4-(4-chloro-2-fluoro-phényl)-3-(3-chloro-2-fluorophényl)-4-cyano-5-(2,2-diméthyl-propyl)-pyrrolidine-2-carbonyl]-amino}-3-méthoxy-benzoïque en une quantité quotidienne d'environ 400 mg/jour à environ 1 600 mg/jour, pendant jusqu'à environ 7 jours, suivie d'une période de repos allant jusqu'à environ 21 jours, ladite administration commençant au premier jour d'un cycle de traitement de 28 jours.

5. Produit pour une utilisation selon la revendication 3, dans lequel la dose d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque consiste en une quantité quotidienne d'environ 400 mg/jour à environ 3 200 mg/jour, pendant jusqu'à environ 5 jours, suivie d'une période de repos allant jusqu'à environ 23 jours, ladite administration commençant au premier jour d'un cycle de traitement de 28 jours.

6. Produit pour une utilisation selon la revendication 3, dans lequel la dose d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque consiste en une quantité d'environ 800 mg/jour à environ 3 200 mg/jour une fois par semaine, suivie d'une période de repos allant jusqu'à environ 21 jours, ladite administration commençant au premier jour d'un cycle de traitement de 28 jours.

7. Produit pour une utilisation selon la revendication 3, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 800 mg/jour à environ 1 500 mg/jour.

8. Produit pour une utilisation selon la revendication 3, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 1 000 mg/jour à environ 2 500 mg/jour.

9. Produit pour une utilisation selon la revendication 3, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré en une quantité d'environ 1 250 mg/jour à environ 1 800 mg/jour.

10. Produit pour une utilisation selon la revendication 3, dans lequel le cycle de traitement est répété tous les 28 jours pendant jusqu'à environ 12 cycles.

**11.** Produit pour une utilisation selon la revendication 3, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré une fois par semaine aux jours 1, 7, 15 d'un cycle hebdomadaire de 28 jours.

**12.** Produit pour une utilisation selon la revendication 3, dans lequel l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque est administré une fois par jour pendant 5 jours par semaine tous les 28 jours.

**13.** Kit comprenant : (a) un premier constituant contenant une ou plusieurs formes galéniques unitaires orales d'un principe actif comprenant de la capécitabine, (b) un deuxième constituant contenant une ou plusieurs formes galéniques unitaires orales d'un principe actif comprenant de l'oxaliplatine et (c) un troisième constituant comprenant de l'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque.

**14.** Kit selon la revendication 13, dans lequel le premier constituant contient un nombre suffisant d'unités de sorte qu'un patient peut se voir administrer d'environ 800 à 1 500 $mg/m^2$ de capécitabine deux fois par jour sur une période de 14 jours et le deuxième constituant contient un nombre suffisant de doses de sorte qu'un patient peut se voir administrer d'environ 75 à 130 $mg/m^2$ toutes les trois semaines et le troisième constituant contient un nombre suffisant de doses de sorte qu'un patient peut se voir administrer d'environ 400 à 3 000 mg par jour d'acide 4-{[(2R,3S,4R,5S)-4-(4-chloro-2-fluoro-phényl)-3-(3-chloro-2-fluoro-phényl)-4-cyano-5-(2,2-diméthyl-propyl)-pyrrolidine-2-carbonyl]-amino}-3-méthoxy-benzoïque pendant une période allant jusqu'à 5 jours.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4966891 A
- US 5472949 A
- US 5453497 A
- US 5476932 A
- US 60532266 B
- WO 2011098398 A
- US 8354444 B
- EP 2014050974 W


### Non-patent literature cited in the description


- **AZMI et al.** *current pharmaceutical design,* 2011, vol. 17, 640-652